# EUROPÄISCHE PATENTSCHRIFT

(11) **EP 2 606 806 B1**
(45) Veröffentlichungstag und Bekanntmachung des Hinweises auf die Patenterteilung: **29.04.2015**
(21) Anmeldenummer: 11401678.5
(22) Anmeldetag: 21.12.2011
(51) Int. Cl.: A47L 15/42, A61L 2/00, B01L 99/00

(54) **Reinigungsgerät mit verminderter Laugenverschleppung**
Cleaning device with reduced lye contamination
Appareil de nettoyage à remorquage de lessive réduit

(43) Veröffentlichungstag der Anmeldung: 26.06.2013
(73) Patentinhaber: Miele & Cie. KG, 33332 Gütersloh (DE)
(72) Erfinder: Knorr, Jochen, 33659 Bielefeld (DE); Kordmöller, Achim, 33607 Bielefeld (DE); Leifeld, Ludger, 59227 Ahlen (DE); Sohnsmeier, Ralf, 32120 Hiddenhausen (DE)

(56) Entgegenhaltungen:
- DE-A1- 3 102 547
- DE-A1-102005 062 480
- DE-A1-102009 009 768
- FR-A1- 2 427 081

## Beschreibung

Die Erfindung betrifft ein Reinigungsgerät, umfassend einen Spülbehälter, einen im Bodenbereich des Spülbehälters angeordneten Sammeltopf, eine in einem Gehäuse angeordnete Umwälzpumpe zur Förderung von Spülflüssigkeit zu mindestens einer Sprüh- oder Reinigungseinrichtung, einen Hauptkanal zur Leitung der Spülflüssigkeit vom Sammeltopf zurück zum Umwälzpumpengehäuse, und einen Nebenkanal zur Spülflüssigkeitsentleerung des Umwälzpumpengehäuses, durch welchen Nebenkanal Spülflüssigkeit vom Umwälzpumpengehäuse unter Umgehung der mindestens einen Sprüh- oder Reinigungseinrichtung und des Spülbehälters in den Sammeltopf gelangen kann.

Reinigungsgeräte, für welche die Erfindung verwendet werden kann, sind Haushaltsgeschirrspüler, gewerbliche Geschirrspüler und Reinigungs- und Desinfektionsautomaten, insbesondere für medizinische Anwendungen, etwa Endoskopspüler und dergleichen.

Derartige Geräte umfassen insbesondere eine Umwälzpumpe in einem zugeordneten Umwälzpumpengehäuse. Mit einer solchen Pumpe wird die Spülflüssigkeit aus dem Spülbehälter, welche im Sammeltopf gesammelt wird, der im Bodenbereich des Spülbehälters angeordnet ist, aus dem Sammeltopf abgepumpt und im Kreislauf über Sprüh- oder Reinigungseinrichtungen des Systems und das Spülgut wieder in den Spülbehälter zurückgeführt. Meist ist die Umwälzpumpe in einer Höhe neben dem Sammeltopf angeordnet und über einen, auch als Ansaugstutzen bezeichneten Hauptkanal, durch den die Spülflüssigkeit aus dem Sammeltopf abgepumpt wird, mit dem Sammeltopf verbunden. Eine solche Umwälzpumpe weist am Pumpengehäuse darüber hinaus zumindest einen Druckstutzen auf, über den eine oder mehrere Sprüh- oder Reinigungseinrichtungen, wie etwa Sprüharme mit Spülflüssigkeit versorgt werden. Reinigungsgeräte dieser Art sind aus dem Stand der Technik an sich bekannt. Eines gesonderten druckschriftlichen Nachweises bedarf es an dieser Stelle deshalb nicht.

Beim Reinigen von Spülgut werden in der Regel Spülprogramme mit mehreren wasserführenden Programmabschnitten, wie z.B. Vorspülen, Reinigen, Nachspülen eingesetzt. Zwischen den Programmabschnitten wird die verschmutzte Spülflüssigkeit typischerweise abgefördert und dem Gerät für den neuen Programmabschnitt frisches Spülwasser zugeführt. Bei den bekannten Geräten verbleibt allerdings ein gewisser Anteil der Spülflotte im Gerät, insbesondere im Umwälzpumpengehäuse, und wird somit in den nachfolgenden Programmabschnitt übernommen, d.h. es kommt in den bekannten Geräten in nicht unerheblichen Maße zu einer sogenannten Laugenverschleppung. Um den nachfolgenden Programmabschnitt jedoch nicht mit Schmutzrückständen und gegebenenfalls chemischen Substanzen des vorangegangenen Programmabschnitts zu belasten, ist es wünschenswert, diese Laugenverschleppung zu verringern. Diese Aufgabe stellt sich in besonderem Maße bei Reinigungs- und Desinfektionsautomaten für medizinische Anwendungen, für die besonders hohe Anforderungen an eine konstant gute Qualität des Spülergebnisses bestehen.

Dies wird bei einem bekannten Reinigungs- und Desinfektionsautomaten der Miele & Cie. KG mit der Bezeichnung PG8528 dadurch gelöst, dass im Gehäuse der Umwälzpumpe ein zusätzlicher Stutzen zur Restentleerung vorgesehen ist. Durch diesen kann die restliche Spülflotte über einen Schlauch unmittelbar zum Sammeltopf geführt werden.

Durch diesen zusätzlichen Ausgang aus der Umwälzpumpe geht jedoch bei Betrieb der Umwälzpumpe stets ein Teil der Pumpenleistung verloren, d.h. es steht nicht die volle Pumpenleistung für deren eigentliche Aufgabe, der Versorgung der Sprüh- oder Reinigungseinrichtungen zur Verfügung. Die Umwälzpumpe arbeitet somit nicht effizient; bei gleicher Leistungsstärke der Umwälzpumpe verringert sich die erreichbare Reinigungsleistung des Geräts. Für eine vorgegebene Reinigungsleistung sind eine leistungsstärkere Umwälzpumpe und/oder ein erhöhter Energieaufwand nötig.

Die DE 31 02 547 A1 beschreibt eine Geschirrspülmaschine mit einer in einem Umwälzpumpengehäuse angeordneter Umwälzpumpe, bei der zur vollständigen Entleerung des Umwälzpumpengehäuses ein Kanal zum Ansaugstutzen der Entleerungspumpe führt, in welchem Kanal ein Rückschlagventil angeordnet ist. Dieses ist bei laufender Umwälzpumpe geschlossen und bei stehender Umwälzpumpe und laufender Entleerungspumpe geöffnet.

Der Erfindung stellt sich somit das Problem, ein Reinigungsgerät bereitzustellen, mit dem bei geringem apparativem Aufwand und ohne Verringerung der Reinigungsleistung oder Vergrößerung des Energieverbrauchs eine Laugenverschleppung minimiert werden kann.

Erfindungsgemäß wird dieses Problem durch ein Reinigungsgerät mit den Merkmalen des Patentanspruchs 1 gelöst. Vorteilhafte Ausgestaltungen und Weiterbildungen der Erfindung ergeben sich aus den nachfolgenden Unteransprüchen.

Das vorgeschlagenen Reinigungsgerät umfasst einen Spülbehälter, einen im Bodenbereich des Spülbehälters angeordneten Sammeltopf, eine in einem Gehäuse angeordnete Umwälzpumpe zur Förderung von Spülflüssigkeit zu mindestens einer Sprüh- oder Reinigungseinrichtung, einen Hauptkanal zur Leitung der Spülflüssigkeit vom Sammeltopf zurück zum Umwälzpumpengehäuse, und einen Nebenkanal zur Spülflüssigkeitsentleerung des Umwälzpumpengehäuses, durch welchen Nebenkanal Spülflüssigkeit vom Umwälzpumpengehäuse unter Umgehung der mindestens einen Sprüh- oder Reinigungseinrichtung und des Spülbehälters, insbesondere unmittelbar, in den Sammeltopf gelangen kann. Dieser Nebenkanal weist darüber hinaus eine Ventileinrichtung auf, die abhängig vom Druck der Nebenkanalströmung, also der Strömung vom Umwälzpumpengehäuse zum Sammeltopf eine Durchlassstellung oder eine Schließstellung einnimmt. Über den Nebenkanal kann somit die nach Abpumpen der Spülflüssigkeit in der Umwälzpumpe verbliebene Restflüssigkeit in den Sammeltopf entlassen werden, von wo sie dann vorzugsweise mittels einer Ablaufpumpe abgefördert wird. Durch die erfindungsgemäße Ventileinrichtung kann der Nebenkanal dabei geschlossen werden, wodurch es möglich ist, einen Durchfluss durch den Nebenkanal nur im Bedarfsfall vorzusehen. Die Einstellung der Ventileinrichtung in eine Durchlassstellung oder eine Schließstellung erfolgt dabei insbesondere in Abhängigkeit des Drucks der Strömung vom Umwälzpumpengehäuse zum Sammeltopf.

Die Einstellung der Ventileinrichtung erfolgt im Übrigen vorzugsweise automatisch, d.h. ohne äußere Einflussnahme. Allein der Druck der Strömung vom Umwälzpumpengehäuse zum Sammeltopf bestimmt, welche Einstellung der Ventileinrichtung eingenommen wird. Da keine zusätzliche Ansteuerung erforderlich bzw. vorhanden ist, ist hierdurch eine besonders einfache Lösung gegeben.

Vorzugsweise wird der Nebenkanal bei Überschreitung eines vorgegebenen Minimaldruckes der Strömung vom Umwälzpumpengehäuse zum Sammeltopf geschlossen, d.h. die Ventileinrichtung nimmt in diesem Fall eine Schließstellung ein. Hierdurch kann der Nebenkanal insbesondere automatisch geschlossen werden und auch geschlossen bleiben, wenn und solange die Umwälzpumpe mit einer bestimmten Leistung in Betrieb genommen wird bzw. in Betrieb ist. Auf diese Weise kann verhindert werden, dass der Nebenkanal während eines Programmabschnittes, in dem die Umwälzpumpe in Betrieb ist, geöffnet ist und dadurch eine Verlustströmung bewirkt.

Außerdem wird der Nebenkanal vorzugsweise bei Unterschreitung eines vorgegebenen Minimaldruckes der Strömung vom Umwälzpumpengehäuse zum Sammeltopf geöffnet, d.h. die Ventileinrichtung nimmt eine Durchlassstellung ein, oder er bleibt gegebenenfalls geöffnet. Insbesondere kann die Ventileinrichtung so ausgebildet sein, dass bei abgestellter Umwälzpumpe der vorgegebene Minimaldruckes der Strömung vom Umwälzpumpengehäuse zum Sammeltopf grundsätzlich nicht erreicht wird, d.h. der durch die im Umwälzpumpengehäuse befindliche Spülflüssigkeit bewirkte Strömungsdruck ist allein, d.h. bei abgestellter Umwälzpumpe nicht ausreichend, um den vorgegebenen Minimaldruck zu erreichen. Die Ventileinrichtung nimmt somit bei abgestellter Umwälzpumpe eine Durchlassstellung ein.

Die Ventileinrichtung des Nebenkanals des erfindungsgemäßen Reinigungsgeräts weist vorzugsweise ein Ventilgehäuse und einen innerhalb der Ventileinrichtung frei beweglichen Schließkörper auf. Das Ventilgehäuse umschließt dabei einen abgesehen von einer ersten und einer zweiten Hohlraumöffnung fluiddichten Hohlraum. Die erste Hohlraumöffnung verbindet den Hohlraum strömungstechnisch über einen ersten Abschnitt des Nebenkanals mit dem Umwälzpumpengehäuse. Die zweite Hohlraumöffnung verbindet den Hohlraum strömungstechnisch entweder gemäß einem ersten Ausführungsbeispiel über einen zum Sammeltopf führenden zweiten Abschnitt des Nebenkanals oder aber gemäß einem zweiten Ausführungsbeispiel unmittelbar mit dem Sammeltopf. Im Falle der Entleerung der Umwälzpumpe bzw. des Umwälzpumpengehäuses gelangt die Restflüssigkeit somit ausgehend vom Umwälzpumpengehäuse durch einen daran angeordneten Entleerungsstutzen, der die Eingangsöffnung des Nebenkanals bildet, in den ersten Abschnitt des Nebenkanals, anschließend in den vom Ventilgehäuse der Ventileinrichtung umfassten Hohlraum und schließlich, entweder über den zweiten Abschnitt des Nebenkanals oder unmittelbar, in den Sammeltopf.

Das Ventilgehäuse umfasst einen Schließkörper, der in dem Hohlraum frei beweglich ist. Der Schließkörper hat solche Abmessungen, dass er den Hohlraum nicht durch die erste oder zweite Hohlraumöffnung verlassen kann. Der Schließkörper und die zweite Hohlraumöffnung sind korrespondierend zueinander darüber hinaus so ausgebildet, dass der Schließkörper eine Schließposition einnehmen kann, in der die zweite Hohlraumöffnung, vorzugsweise fluiddicht oder zumindest im Wesentlichen fluiddicht, geschlossen wird, so dass die Ventileinrichtung eine Schließstellung einnimmt. Der Schließkörper kann andererseits an einer von der ersten und zweiten Hohlraumöffnung beabstandeten Stelle des Hohlraums eine Durchlassposition einnehmen, wobei die Ventileinrichtung in Durchlassstellung gebracht ist, die Spülflüssigkeit also die Ventileinrichtung durchfließen kann. Der Hohlraum ist so geformt, dass der Schließkörper an relativ zur Schließposition tiefer gelegenen Stellen des Hohlraums eine Durchlassposition einnehmen kann; insbesondere an der tiefst gelegenen, vorzugsweise zwischen der ersten und zweiten Hohlraumöffnung angeordneten Stelle des Hohlraums befindet sich der Schließkörper in Durchlassposition. Der Schließkörper wird somit durch seine Schwerkraft in eine Durchlassposition gedrängt. Auf diese Weise wird auf besonders einfache Weise sichergestellt, dass die Ventileinrichtung automatisch eine Durchlassstellung einnimmt oder darin verharrt, wenn ein vorgegebener Minimaldruck der Strömung vom Umwälzpumpengehäuse zum Sammeltopf nicht erreicht oder unterschritten wird. Dabei ist die Masse des Schließkörpers vorzugsweise so gewählt, dass der Schließkörper bei Überschreitung des vorgegebenen Minimaldruckes der Nebenkanalströmung vom Umwälzpumpengehäuse zum Sammeltopf von der Strömung aus einer Durchlassposition, insbesondere der tiefst gelegenen Stelle des Hohlraums, entgegen der Wirkung seiner Schwerkraft in die Schließposition getrieben wird.

Vorzugsweise ist der Schließkörper eine Kugel, insbesondere eine Stahlkugel. Entsprechend hat die zweite Hohlraumöffnung vorzugsweise einen runden Querschnitt, dessen Durchmesser kleiner ist als der Durchmesser des kugelförmigen Schließkörpers. Ein geeigneter Kugeldurchmesser liegt im Bereich von 5 bis 30 mm, vorzugsweise zwischen 10 und 20 mm. Das Gewicht des Schließkörpers, insbesondere der Kugel, liegt zwischen 5 und 25 g, vorzugsweise zwischen 10 und 20 g.

Der Bodenbereich des Ventilgehäuses ist vorzugsweise so geformt, dass von der tiefst gelegenen Stelle zwischen den beiden Hohlraumöffnungen zur Schließposition vor der zweiten Hohlraumöffnung ein stetiger Anstieg vorliegt. Die Größe und Form des Hohlraums und die Größe des Schließkörpers, sind so aufeinander abgestimmt, dass der Schließkörper in jeder Position des Hohlraums ein Strömungshindernis, insbesondere ein wesentliches Strömungshindernis darstellt. Insbesondere ist die maximale Abmessung des Schließkörpers, insbesondere der Durchmesser der Kugel, vorzugsweise größer als die Hälfte der maximalen Abmessung des Hohlraums quer zur Strömungsrichtung. Die Querschnittsfläche des Hohlraums quer zur Strömungsrichtung variiert entlang des Wegs zwischen erster Hohlraumöffnung, größter Aufweitung des Hohlraums zwischen den Hohlraumöffnungen und zweiter Hohlraumöffnung; sie ist jedoch an jeder Stelle zwischen den beiden Hohlraumöffnungen höchstens viermal, vorzugsweise höchstens zweimal so groß wie die maximale Querschnittsfläche des Schließkörpers, vorzugsweise der Kugel.

Ein besonderer Vorteil der Ventileinrichtung des erfindungsgemäßen Reinigungsgeräts besteht offensichtlich darin, dass die Ventileinrichtung bei einem bestimmten ersten Druckbereich der Strömung vom Umwälzpumpengehäuse zum Sammeltopf den Nebenkanal schließt oder geschlossen hält, während sie bei einem zweiten Druckbereich der Strömung vom Umwälzpumpengehäuse zum Sammeltopf den Nebenkanal öffnet oder offen lässt. Die Einstellung der Ventileinrichtung erfolgt somit nicht durch die Strömungsrichtung, sondern durch den Druck der Strömung aus lediglich einer Strömungsrichtung.

Um eine bestmögliche Entleerung der Umwälzpumpe von der Restflüssigkeit zu erreichen, weist der Nebenkanal eine Eingangsöffnung nahe dem tiefst gelegenen Punkt oder am tiefst gelegenen Punkt des Umwälzpumpengehäuses auf. Darüber hinaus hat der Nebenkanal eine Ausgangsöffnung im Sammeltopf. Diese Ausgangsöffnung befindet sich vorzugsweise im unteren Bereich des Sammeltopfs, d.h. am oder nahe dem tiefsten Punkt des Spülsystems, insbesondere nahe dem und/oder etwa auf einer Höhe mit dem Ausgangsstutzen des Sammeltopfs zur Ablaufpumpe bzw. dem Ablaufventil. Dieser Bereich sollte ein spültechnisch beruhigter Bereich sein, da sich in diesem Bereich Schmutzreste ablagern können bzw. sollen, womit sie bereits aus dem Spülkreislauf ausscheiden und am Ende des Programmabschnitts abgefördert werden können. Mit einem Nebenkanal ohne die Ventileinrichtung entsprechend der Erfindung besteht die Gefahr, dass in diesem Bereich befindliche Schmutzreste durch die Strömung aus dem Nebenkanal aufgewirbelt werden und dadurch wieder in den Spülkreislauf und damit in den Spülraum geraten, bzw. das stark verschmutzte Spülflotte in höher gelegene Bereiche des Sammeltopfs und schließlich in den Spülraum zurückströmt. Dieser Nachteil bzw. diese Gefahr wird bei dem erfindungsgemäßen Reinigungsgerät mit der Ventileinrichtung vermieden, dadurch dass durch die Ausbildung der Ventileinrichtung sichergestellt ist, dass ein vollständiges Durchströmen des Nebenkanals bis in den Sammeltopf hinein vorzugsweise nur beim Ablaufen des Restwassers aus dem Umwälzpumpengehäuse erfolgt, jedoch nicht im Spülbetrieb bei laufender Umwälzpumpe.

In einem ersten Ausführungsbeispiel umfasst der Nebenkanal zwei Kanalabschnitte, deren einander zugewandte Endbereiche gemeinsam das Ventilgehäuse der Ventileinrichtung bilden. Anders ausgedrückt ist die Ventileinrichtung gemäß diesem Ausführungsbeispiel strömungstechnisch zwischen einem ersten und einem zweiten Kanalabschnitt des Nebenkanals angeordnet. Vorzugsweise sind die zugewandten Endbereiche der beiden Kanalabschnitte lösbar miteinander verbunden. Dies erlaubt eine einfache Montage, Demontage und Reinigung der Ventileinrichtung. Die Ventileinrichtung besteht bei diesem Ausführungsbeispiel in besonders einfacher Weise lediglich aus den Endbereichen der beiden Kanalabschnitte sowie dem innerhalb des durch die Endbereiche gebildeten Hohlraums einzusetzenden Schließkörper, also beispielsweise der Stahlkugel. Vorzugsweise werden dabei ein Endbereich oder beide Endbereiche einfach durch im Querschnitt aufgeweitete Bereiche des Nebenkanals gebildet. Durch diese aufgeweiteten Bereiche des Nebenkanals wird der Hohlraum gebildet.

In einem besonders vorteilhaften alternativen Ausführungsbeispiel ist die Ventileinrichtung dagegen am Ende des Nebenkanals und damit unmittelbar vor der Öffnung in den Sammeltopf angeordnet. An die zweite Hohlraumöffnung schließt sich somit bei diesem Ausführungsbeispiel kein weiterer Abschnitt des Nebenkanals an, stattdessen stellt die zweite Hohlraumöffnung gleichzeitig die Ausgangsöffnung des Nebenkanals in den Sammeltopf dar. Erfindungsgemäß ist zumindest ein Teil des Ventilgehäuses in einem entnehmbaren Einsatz des Sammeltopfs angeordnet. Der Benutzer kann somit auf einfache Weise über den vergleichsweise leicht erreichbaren Sammeltopf an die Ventileinrichtung gelangen. Auf diese Weise ist die Montage, Demontage und Reinigung der Ventileinrichtung für den Benutzer besonders einfach. Vorteilhafterweise wird durch Entnehmen des Einsatzes das Innere der Ventileinrichtung, insbesondere der Schließkörper für den Benutzer frei zugänglich. Beim Einsetzen des Einsatzes verrastet der Einsatz im Sammeltopf formschlüssig.

Ein Ausführungsbeispiel der Erfindung ist in den Zeichnungen rein schematisch dargestellt und wird nachfolgend näher beschrieben. Es zeigt
- Figur 1: einen Ausschnitt eines Reinigungsgeräts gemäß einem ersten, nicht beanspruchten Ausführungsbeispiel
- Figur 2: eine Ventileinrichtung in Durchlassstellung gemäß einem nicht beanspruchten Ausführungsbeispiel
- Figur 3: eine Ventileinrichtung in Schließstellung gemäß einem nicht beanspruchten Ausführungsbeispiel
- Figur 4: einen schematischen Ausschnitt eines Sammeltopfes mit Ventileinrichtung in Schließstellung gemäß einem Ausführungsbeispiel der Erfindung
- Figur 5: einen schematischen Ausschnitt eines Sammeltopfes mit Ventileinrichtung in Durchlassstellung gemäß einem Ausführungsbeispiel der Erfindung

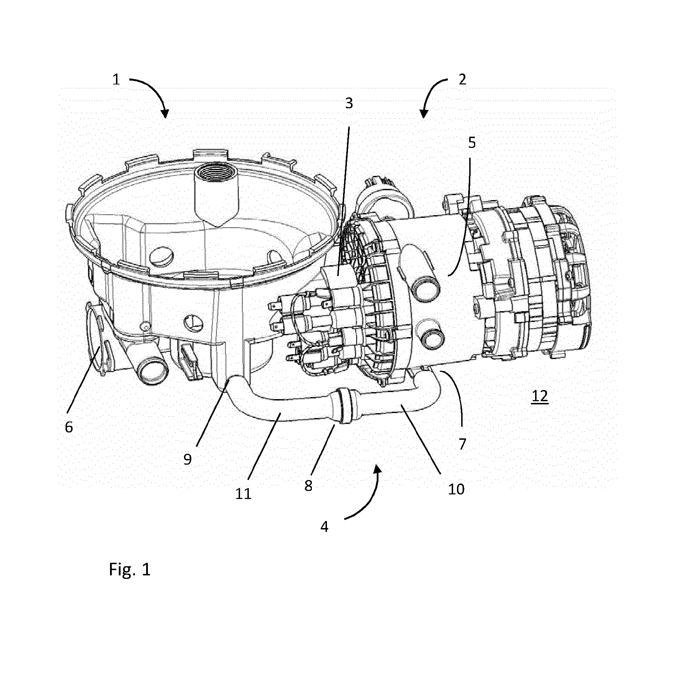

Figur 1 zeigt einen Ausschnitt eines Reinigungsgeräts gemäß einem nicht beanspruchten Ausführungsbeispiel mit einem Sammeltopf 1 und einem damit über einen Hauptkanal 3 verbundenen Umwälzpumpengehäuse 2. Das Umwälzpumpengehäuse weist in bekannter Weise einen Ausgangstutzen 5 auf zur Leitung von Spülflüssigkeit zu mindestens einer, nicht gezeigten, Sprüh- oder Reinigungseinrichtung, typischerweise zu mehreren solcher Einrichtungen. Der Sammeltopf 1 umfasst ebenfalls in bekannter Weise einen Verbindungsstutzen 6 zu einer nicht gezeigten Ablaufpumpe. Zwischen dem Sammeltopf 1 und dem Umwälzpumpengehäuse 2 ist darüber hinaus ein Nebenkanal 4 angeordnet. Dieser ist an oder nahe der tiefst gelegenen Stelle des Umwälzpumpengehäuse 2 an einen Entleerungsstutzen 7 des Umwälzpumpengehäuses 2 angeschlossen. Mit dem Sammeltopf 1 ist der Nebenkanal 4 über einen im unteren Bereich des Sammeltopfs 1 angeordneten Einlassstutzen 9 verbunden. Der Nebenkanal 8 weist einen ersten Kanalabschnitt 10 und einen zweiten Kanalabschnitt 11 auf. Zwischen den beiden Kanalabschnitten 10,11 ist eine Ventileinrichtung 8 angeordnet.

Im bestimmungsgemäßen Gebrauch saugt die Umwälzpumpe 12 die Spülflüssigkeit vom Sammeltopf 1 durch den Hauptkanal 3 an und fördert diese aus dem Ausgangsstutzen 5 hinaus zu einer oder mehreren nicht gezeigten Sprüh- oder Reinigungseinrichtungen, durch die das im Spülbehälter befindliche Spülgut mit der Spülflüssigkeit beaufschlagt wird. Anschließend gelangt die Spülflüssigkeit aus dem Spülbehälter wieder in den Sammeltopf 1. Nach einem Programmabschnitt wird die Spülflüssigkeit aus dem Sammeltopf 1 mittels der Ablaufpumpe aus dem Reinigungsgerät abgefördert. Im Umwälzpumpengehäuse 2 übrig bleibende Restflüssigkeit gelangt, typischerweise bei ausgestellter Umwälzpumpe 12 durch den Nebenkanal 4 ebenfalls in den Sammeltopf 1, von wo es abgefördert werden kann.

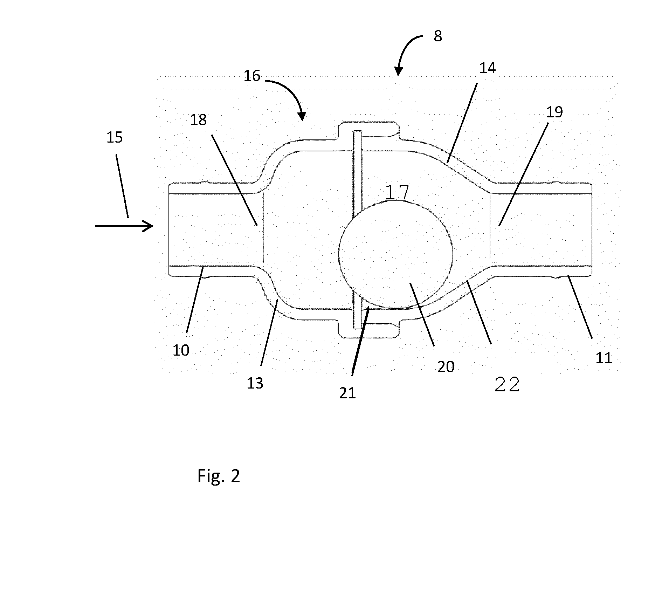

In Fig. 2 ist ein Ausschnitt des Nebenkanals 4 mit der Ventileinrichtung 8 gemäß einem nicht beanspruchten Ausführungsbeispiel entsprechend Fig. 1 im Längsschnitt schematisch dargestellt. Zwischen den beiden Kanalabschnitten 10,11 des Nebenkanals 4 ist die Ventileinrichtung 8 angeordnet. Die beiden Kanalabschnitte 10,11 weisen einander zugewandte Endbereiche 13,14 auf. In diesen Endbereichen 13,14 weist der Nebenkanal 4 jeweils eine Aufweitung seines Querschnitts quer zur Strömungsrichtung 15 auf. Die aufgeweiteten Endbereiche 13,14 bilden gemeinsam das Ventilgehäuse 16 der Ventileinrichtung 8. Dieses umschließt einen Hohlraum 17 mit einer ersten Hohlraumöffnung 18 und einer zweiten Hohlraumöffnung 19. Die aufgeweiteten Endbereiche 13,14 sind abdichtend und lösbar miteinander verbunden, wofür sie in an sich bekannter Weise Verbindungs- und Dichtungsmittel umfassen, wie etwa Gewinde, O-Ring und dergleichen. Im Hohlraum 17 der Ventileinrichtung 8 befindet sich ein Schließkörper 20, vorzugsweise eine Kugel aus Stahl, insbesondere mit einem Durchmesser von 15mm und einer Masse von 14 g. Der Schließkörper 20 ist frei im Hohlraum 17 beweglich. Er bewegt sich innerhalb des Hohlraums 17 abhängig von dem Druck der Strömung durch den Nebenkanal 4. Da sein Durchmesser größer ist als die der beiden Hohlraumöffnungen 18,19, kann er diese nicht durchqueren und den Hohlraum 17 daher auf diese Weise nicht verlassen. In Fig. 2 befindet sich die Verstelleinrichtung 8 in Durchlassstellung, bei dem der Schließkörper 20 eine Durchlassposition eingenommen hat. Hierfür weist der Hohlraum 17 eine durch die Aufweitung der Endbereiche 13,14 der Kanalabschnitte 10,11 gebildete Vertiefung 21, welche niedriger gelegen ist als der Nebenkanal 4 bzw. die beiden Hohlraumöffnungen 18,19. Der Schließkörper 20, der diese Durchlassposition in der Vertiefung 21 eingenommen hat, weist zwar einen Querschnitt auf, der einen wesentlichen Teil des Querschnitts des Hohlraums 17 quer zur Strömungsrichtung 15 abdeckt, so ist im gezeigten Beispiel der Durchmesser der kugelförmigen Schließkörpers 20 zumindest größer als der Radius der maximalen Querschnittsfläche des Hohlraums 17. Die Spülflüssigkeit hat jedoch noch genügend Platz im Hohlraum 17, um am Schließkörper 20 vorbei die Verstelleinrichtung 8 zu durchströmen. Bei lediglich geringem Druck der Strömung ist diese nicht in der Lage, den Schließkörper 20 entgegen der Schwerkraft des Schließkörpers 20 zu der zweiten Hohlraumöffnung 19 zu bewegen, wofür die von der Vertiefung 21 zur Hohlraumöffnung 19 ansteigende Rampe 22 zu überwinden ist.

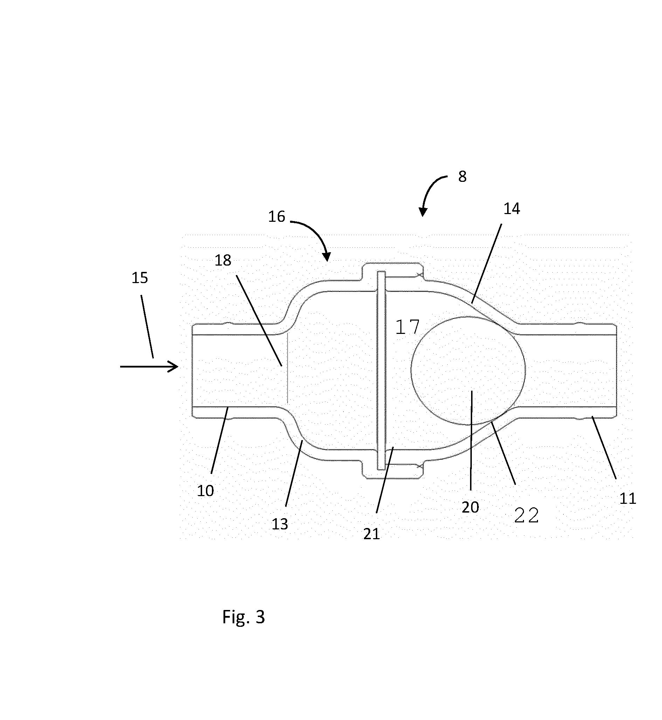

Bei hinreichendem Druck der Strömung vom Umwälzpumpengehäuse 2 zum Sammeltopf 1 wird der Schließkörper 20 dagegen in Richtung der Hohlraumöffnung 19 getrieben, bis er diese verschließt, wobei er nahe der Hohlraumöffnung 19 eine Schließposition einnimmt, wie in Fig. 3 gezeigt. Die Verstelleinrichtung 8 ist damit in Schließstellung, wobei keine oder nahezu keine weitere Flüssigkeit durch den Nebenkanal 4 in den Sammeltopf 1 gelangt.

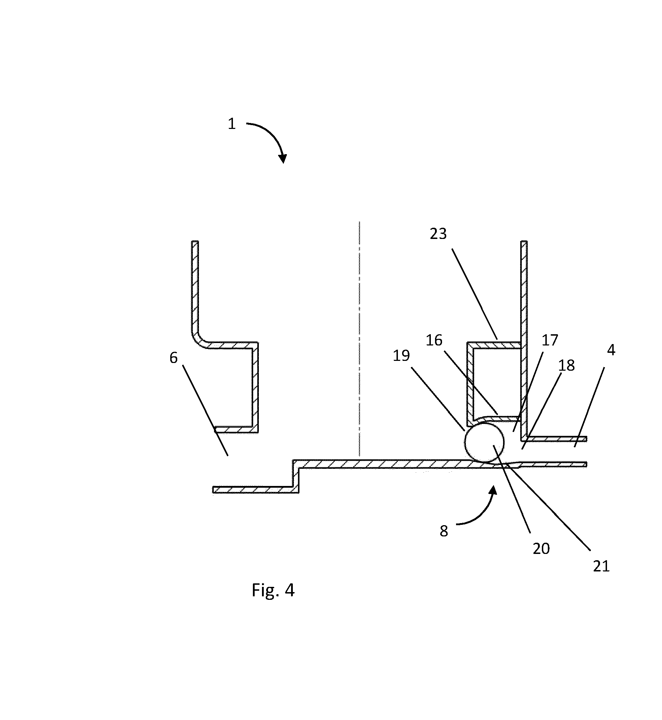

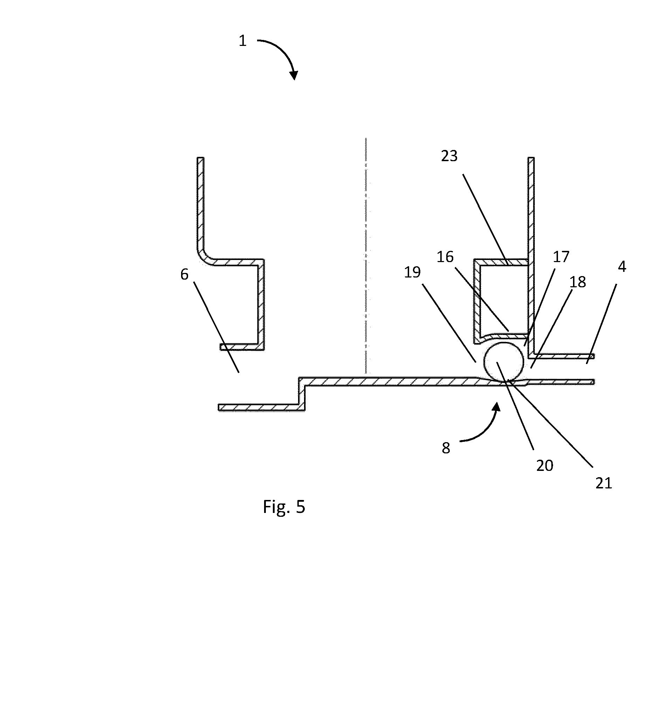

Fig. 4 und Fig. 5 zeigen schematisch einen Ausschnitt des Sammeltopfs 1 gemäß einem Ausführungsbeispiel des erfindungsgemäßen Reinigungsgeräts. Dabei ist die Ventileinrichtung 8 mit in den Sammeltopf 1 integriert. Der Nebenkanal 4 schließt mit der Ventileinrichtung 8 ab. Die zweite Hohlraumöffnung 19 stellt unmittelbar die Einlassöffnung in den Sammeltopf 1 dar, d.h. es ist kein zweiter Nebenkanalabschnitt 11 zwischengeordnet wie im Ausführungsbeispiel gemäß Fig. 1 bis 3. Im Übrigen entspricht diese Ausführungsform in Ihrer Wirkungsweise jedoch dem Ausführungsbeispiel gemäß Fig. 1 bis 3. In Fig. 4 befindet sich die Ventileinrichtung 8 in Schließstellung, in der der als Kugel ausgestaltete Schließkörper 20 nahe der Hohlraumöffnung 19 eine Schließposition einnimmt. In Fig. 5 befindet sich die Ventileinrichtung 8 dagegen in Durchlassstellung, in der der als Kugel ausgestaltete Schließkörper 20 in einer Vertiefung 21 eine Durchlassposition einnimmt. Die zweite Hohlraumöffnung 19 bzw. die Einlassöffnung in den Sammeltopf 1 befindet sich im unteren Bereich des Sammeltopfs 1, insbesondere in Höhenrichtung allenfalls nur wenig oberhalb des Bodens des Sammeltopfs und des Verbindungsstutzen 6 zur Ablaufpumpe. Das Ausführungsbeispiel gemäß Fig. 4 und Fig. 5 zeichnet sich vor allem dadurch aus, dass der Sammeltopf 1 einen aus dem Sammeltopf 1 entnehmbaren Einsatz 23 aufweist. Dieser Einsatz 23 umfasst einen Teil des Ventilgehäuses 16 der Ventileinrichtung 8, nämlich nach oben hin den Hohlraum 17 abdeckende Bereiche und/oder die zweite Hohlraumöffnung 19 umschließende Bereiche des Ventilgehäuses 16. Wird der Einsatz 23 aus dem Sammeltopf entnommen, so hat der Benutzer Zugang zu dem Hohlraum 17 und dem darin befindlichem Schließkörper 20 der Ventileinrichtung 8 und kann einzelne Elemente 18,19,20,21,22 der Ventileinrichtung 8 prüfen, säubern oder ersetzen und insbesondere den Schließkörper 20 entnehmen.

### Bezugszeichenliste

- 1: Sammeltopf
- 2: Umwälzpumpengehäuse
- 3: Hauptkanal
- 4: Nebenkanal
- 5: Ausgangstutzen zu Sprüheinrichtung
- 6: Verbindungsstutzen zu Ablaufpumpe
- 7: Entleerungsstutzen
- 8: Ventileinrichtung
- 9: Einlassstutzen für den Nebenkanal in den Sammeltopf
- 10: Erster Nebenkanalabschnitt
- 11: Zweiter Nebenkanalabschnitt
- 12: Umwälzpumpe
- 13: Endbereich des ersten Nebenkanalabschnitts
- 14: Endbereich des zweiten Nebenkanalabschnitts
- 15: Strömungsrichtung
- 16: Ventilgehäuse
- 17: Hohlraum
- 18: Erste Hohlraumöffnung
- 19: Zweite Hohlraumöffnung
- 20: Schließkörper
- 21: Vertiefung
- 22: Rampe
- 23: Einsatz

## Patentansprüche

1. Reinigungsgerät, umfassend einen Spülbehälter, einen im Bodenbereich des Spülbehälters angeordneten Sammeltopf (1), eine in einem Gehäuse (2) angeordnete Umwälzpumpe zur Förderung von Spülflüssigkeit zu mindestens einer Sprüh- oder Reinigungseinrichtung, einen Hauptkanal (3) zur Leitung der Spülflüssigkeit vom Sammeltopf (1) zurück zum Umwälzpumpengehäuse (2), und einen Nebenkanal (4) zur Spülflüssigkeitsentleerung des Umwälzpumpengehäuses (2), durch welchen Nebenkanal (4) Spülflüssigkeit vom Umwälzpumpengehäuse (2) unter Umgehung der mindestens einen Sprüh- oder Reinigungseinrichtung und des Spülbehälters in den Sammeltopf (1) gelangen kann, wobei der Nebenkanal (4) eine Ventileinrichtung (8) aufweist, die abhängig vom Druck der Strömung vom Umwälzpumpengehäuse (2) zum Sammeltopf (1) eine Durchlassstellung oder eine Schließstellung einnimmt, **dadurch gekennzeichnet, dass** die Ventileinrichtung (8) ein Ventilgehäuse (16) aufweist, wobei zumindest ein Teil des Ventilgehäuses (16) in einem entnehmbaren Einsatz (23) des Sammeltopfs (1) angeordnet ist, so dass das Innere der Ventileinrichtung (8) bei Entnehmen des Einsatzes (23) durch den Bediener frei zugänglich ist.

2. Reinigungsgerät nach Anspruch 1, **dadurch gekennzeichnet, dass** der Nebenkanal (4) bei Überschreitung eines vorgegebenen Minimaldruckes der Strömung vom Umwälzpumpengehäuse (2) zum Sammeltopf (1) geschlossen wird.

3. Reinigungsgerät nach Anspruch 1, **dadurch gekennzeichnet, dass** die Ventileinrichtung (8) einen innerhalb der Ventileinrichtung (8) frei beweglichen Schließkörper (20) aufweist, welcher durch seine Schwerkraft in eine Durchlassposition gedrängt wird.

4. Reinigungsgerät nach einem der vorhergehenden Ansprüche, **dadurch gekennzeichnet, dass** die Ventileinrichtung (8) bei abgestellter Umwälzpumpe (12) eine Durchlassstellung einnimmt.

5. Reinigungsgerät nach einem der vorhergehenden Ansprüche, **dadurch gekennzeichnet, dass** der Nebenkanal (4) eine Eingangsöffnung (7) nahe des tiefst gelegenen Punktes des Umwälzpumpengehäuses (2) und eine Ausgangsöffnung im Sammeltopf (1) aufweist.

6. Reinigungsgerät nach einem der vorhergehenden Ansprüche, **dadurch gekennzeichnet, dass** die Ventileinrichtung (8) unmittelbar vor der Ausgangsöffnung des Nebenkanals (4) angeordnet ist.

## Claims

1. Cleaning device comprising a washing container, a collecting vessel (1) arranged at the bottom of the washing container, a recirculating pump arranged in a housing (2) and intended for conveying washing fluid to at least one spraying or cleaning apparatus, a main conduit (3) for conducting the washing fluid from the collecting vessel (1) back to the recirculating pump housing (2), and an auxiliary conduit (4) for draining washing fluid from the recirculating pump housing (2), said auxiliary conduit (4) enabling washing fluid from the recirculating pump housing (2) to reach the collecting vessel (1) by bypassing both the at least one spraying or cleaning apparatus and the washing container, the auxiliary conduit (4) comprising a valve apparatus (8) which assumes an open position or a closed position depending on the pressure of the flow from the recirculating pump housing (2) to the collecting vessel (1), **characterised in that** the valve apparatus (8) comprises a valve housing (16), at least part of the valve housing (16) being arranged in a removable insert (23) of the collecting vessel (1), and therefore the inner space of the valve apparatus (8) can be accessed by the operator when the insert (23) has been removed.

2. Cleaning device according to claim 1, **characterised in that** the auxiliary conduit (4) is closed if a predetermined minimum pressure in the flow from the recirculating pump housing (2) to the collecting vessel (1) is exceeded.

3. Cleaning device according to claim 1, **characterised in that** the valve apparatus (8) comprises a closing body (20) which can move freely inside the valve apparatus (8) and is urged into an open position by means of the gravitational force thereof.

4. Cleaning device according to any of the preceding claims, **characterised in that** the valve apparatus (8) assumes an open position when the recirculating pump (12) is switched off.

5. Cleaning device according to any of the preceding claims, **characterised in that** the auxiliary conduit (4) has an inlet opening (7) near to the lowest point of the recirculating pump housing (2) and an outlet opening in the collecting vessel (1).

6. Cleaning device according to any of the preceding claims, **characterised in that** the valve apparatus (8) is arranged directly in front of the outlet opening of the auxiliary conduit (4).

## Revendications

1. Appareil de nettoyage, comprenant une cuve de lavage, un bac collecteur (1) disposé dans la zone de fond de la cuve de lavage, une pompe de recirculation disposée dans un carter (2) pour transporter le liquide de lavage vers au moins un dispositif de pulvérisation ou de nettoyage, un canal principal (3) pour ramener le liquide de lavage à partir du bac collecteur (1) vers le carter (2) de pompe de recirculation, et un canal secondaire (4) pour vidanger le liquide de lavage hors du carter (2) de pompe de recirculation, canal secondaire (4) à travers lequel le liquide de lavage peut quitter le carter (2) de pompe de recirculation pour gagner le bac collecteur (1) en contournant le dispositif de pulvérisation ou de nettoyage au moins au nombre de un et la cuve de lavage, le canal secondaire (4) présentant un dispositif de vanne (8) qui, en fonction de la pression de l'écoulement provenant du carter (2) de pompe de recirculation et dirigé vers le bac collecteur (1), adopte une position de passage ou une position de fermeture, **caractérisé en ce que** le dispositif de vanne (8) présente un carter de vanne (16), au moins une partie du carter de vanne (16) étant disposée dans un insert (23) amovible du bac collecteur (1) de telle sorte que l'intérieur du dispositif de vanne (8) est librement accessible à l'utilisateur lors de la dépose de l'insert (23).

2. Appareil de nettoyage selon la revendication 1, **caractérisé en ce que** le canal secondaire (4) est fermé en cas de dépassement d'une pression minimale prédéfinie de l'écoulement provenant du carter (2) de pompe de recirculation et dirigé vers le bac collecteur (1).

3. Appareil de nettoyage selon la revendication 1, **caractérisé en ce que** le dispositif de vanne (8) présente un corps de fermeture (20), librement mobile à l'intérieur du dispositif de vanne (8), qui est poussé dans une position de passage par sa force de gravité.

4. Appareil de nettoyage selon l'une des revendications précédentes, **caractérisé en ce que** le dispositif de vanne (8) adopte une position de passage quand la pompe de recirculation (12) est à l'arrêt.

5. Appareil de nettoyage selon l'une des revendications précédentes, **caractérisé en ce que** le canal secondaire (4) présente une ouverture d'entrée (7) près du point le plus bas du carter (2) de pompe de recirculation et une ouverture de sortie dans le bac collecteur (1).

6. Appareil de nettoyage selon l'une des revendications précédentes, **caractérisé en ce que** le dispositif de vanne (8) est disposé immédiatement devant l'ouverture de sortie du canal secondaire (4).
